# EUROPEAN PATENT APPLICATION

(11) **EP 2 889 790 A1**
(43) Date of publication of application: **01.07.2015**
(21) Application number: 14200054.6
(22) Date of filing: 23.12.2014
(51) Int. Cl.: G06F 19/00

(54) **Vital information measuring device, processing system, vital information processing method, and program**

(30) Priority: 27.12.2013 JP 2013271207
(71) Applicant: Seiko Epson Corporation, Tokyo 163 (JP)
(72) Inventor: Saito, Toshiki, Nagano, 392-8502 (JP)
(74) Representative: Hoffmann Eitle

(57) **Abstract**

A vital information measuring device includes: a power state information acquiring unit that acquires power state information indicating a power state; a vital information acquiring unit that acquires a plurality of pieces of vital information from a plurality of vital sensors; and a processing unit that performs power management of the plurality of vital sensors based on the power state information, wherein the processing unit sets a priority of power supply for each of the plurality of vital sensors based on each of the plurality of pieces of vital information, and executes the power management on the plurality of vital sensors based on the priority and the power state information.

## Description

### BACKGROUND

### 1. Technical Field

The present invention relates to a vital information measuring device, a processing system, a vital information processing method, a program, and the like.

### 2. Related Art

Portable vital information measuring devices that include a vital sensor mounted thereon and constantly measure and analyze vital information are increasing. Especially in recent years, devices that include a plurality of vital sensors mounted thereon and can collect more vital information have emerged, and it is expected that the number of vital sensors to be mounted will increase more and more in the future.

In such a portable and constant measuring vital information measuring device, proper power management is important. For example, JP-A-2006-320735 proposes a power management technique in which message display for a user is turned off in sleeping, or a specific sensor is turned off in sleeping.

As described above, the number of vital sensors to be mounted on the vital information measuring device tends to increase, whereas vital information in which a user (or the persons concerned such as the user's doctor or family) using the vital information measuring device is really interested is limited only to a portion of various pieces of acquired vital information in many cases.

On the other hand, the technique proposed in JP-A-2006-320735 is to turn off a specific sensor in sleeping as described above, in which power management in terms of how important each vital sensor is for the user (how high a priority is) is not performed. Therefore, a technique in which the priority is determined based on the past vital information is not also disclosed.

### SUMMARY

An aspect of the invention relates to a vital information measuring device including: a power state information acquiring unit that acquires power state information indicating a power state; a vital information acquiring unit that acquires a plurality of pieces of vital information from a plurality of vital sensors; and a processing unit that performs power management of the plurality of vital sensors based on the power state information, wherein the processing unit sets a priority of power supply for each of the plurality of vital sensors based on each of the plurality of pieces of vital information, and executes the power management on the plurality of vital sensors based on the priority and the power state information.

In the aspect of the invention, the priority of power supply is set for each of the vital sensors based on each piece of the vital information, and the power management is executed based on the priority and the power state information. Therefore, it is possible to set the priority of the vital sensor based on the measured vital information and perform the power management based on the priority, so that, for example, proper power management can be executed on each of the sensors.

In the aspect of the invention, the processing unit may perform, based on the plurality of pieces of vital information, a process of determining whether or not an abnormal value is detected in the plurality of vital sensors, and when the abnormal value is detected in a first vital sensor and the abnormal value is not detected in a second vital sensor that is different from the first sensor in the plurality of vital sensors, the processing unit may set the priority of the first vital sensor higher than the priority of the second vital sensor.

With this configuration, for example, the priority can be set based on the determination of the detection or non-detection of the abnormal value.

In the aspect of the invention, when it is determined based on the power state information that remaining battery power is equal to or lower than a predetermined value, the processing unit may perform the power management in which the vital sensor whose priority is high is set into a normal power consumption mode and the vital sensor whose priority is low is set into a low power consumption mode or an off state.

With this configuration, for example, proper power management according to the priority can be performed when the remaining battery power is reduced.

In the aspect of the invention, when, in the plurality of vital sensors, a first priority is set for a first vital sensor, a second priority that is low compared to the first priority is set for a second vital sensor, and a third priority that is low compared to the second priority is set for a third vital sensor, the processing unit may set the first vital sensor, the second vital sensor, and the third vital sensor into a normal power consumption mode in a first power state, set the first vital sensor and the second vital sensor into the normal power consumption mode and set the third vital sensor into a low power consumption mode or an off state in a second power state in which remaining battery power is low compared to the first power state, and set the first vital sensor into the normal power consumption mode and set the second vital sensor and the third vital sensor into the low power consumption mode or the off state in a third power state in which the remaining battery power is low compared to the second power state.

With this configuration, for example, the power management can be performed by dividing each of the remaining battery power and the priority into three or more levels.

In the aspect of the invention, the processing unit may set a threshold value of the priority based on the power state information, set the vital sensor whose set priority is higher than the threshold value in the plurality of vital sensors into a normal power consumption mode, and set the vital sensor whose set priority is equal to or lower than the threshold value in the plurality of vital sensors into a low power consumption mode or an off state.

With this configuration, for example, the power management can be performed by setting the threshold value based on the power state information and performing a process of comparing the threshold value with the priority of each of the vital sensors.

Another aspect of the invention relates to a processing system including: a receiving unit that receives a plurality of pieces of vital information from a plurality of vital sensors provided in a vital information measuring device; a processing unit that produces priority information used for power management of the vital information measuring device; and a transmitting unit that transmits the priority information to the vital information measuring device, wherein the processing unit generates, based on each of the plurality of pieces of vital information, the priority information for setting a priority of power supply for each of the plurality of vital sensors.

In the aspect of the invention, the priority information used for the power management of the vital information measuring device can be set by receiving and analyzing the vital information. Therefore, for example, the processes relating to the power management described above can be executed with a device other than the vital information measuring device.

Still another aspect of the invention relates to a vital information processing method including: receiving a plurality of pieces of vital information from a plurality of vital sensors provided in a vital information measuring device; generating, based on each of the plurality of pieces of vital information, priority information used for power management of the vital information measuring device and for setting a priority of power supply for each of the plurality of vital sensors; and transmitting the priority information to the vital information measuring device.

In the aspect of the invention, the priority information used for the power management of the vital information measuring device can be set by receiving and analyzing the vital information.

Yet another aspect of the invention relates to a program causing a computer to function as: a receiving unit that receives a plurality of pieces of vital information from a plurality of vital sensors provided in a vital information measuring device; a processing unit that produces priority information used for power management of the vital information measuring device; and a transmitting unit that transmits the priority information to the vital information measuring device, wherein the processing unit generates, based on each of the plurality of pieces of vital information, the priority information for setting a priority of power supply for each of the plurality of vital sensors.

In the aspect of the invention, it is possible to cause a computer to execute the process of setting the priority information used for the power management of the vital information measuring device by receiving and analyzing the vital information.

According to some aspects of the invention as described above, it is possible to provide a vital information measuring device in which a priority is set for each of vital sensors based on acquired vital information to thereby perform power management corresponding to the measured result of the vital information, a processing system, a vital information processing method, a program, and the like.

### BRIEF DESCRIPTION OF THE DRAWINGS

The invention will be described with reference to the accompanying drawings, wherein like numbers reference like elements.
Fig. 1 shows a configuration example of a vital information measuring device according to an embodiment.
Fig. 2 shows a detailed configuration example of the vital information measuring device according to the embodiment.
Figs. 3A and 3B each show a specific example of the vital information measuring device.
Fig. 4 shows a device configuration example of the vital information measuring device according to the embodiment.
Fig. 5 is a functional block diagram of the vital information measuring device according to the embodiment.
Fig. 6 shows a configuration example of a processing system according to the embodiment.
Fig. 7 shows an example of an information processing system including the processing system according to the embodiment.
Figs. 8A and 8B are flowcharts explaining processes of the embodiment.
Figs. 9A and 9B each are a relationship diagram between vital information and priorities.
Fig. 10 shows an example of sensor control using information of priorities and power state information.
Fig. 11 shows an example of setting the threshold value of priority based on remaining battery power.
Fig. 12 shows other flowcharts explaining processes of the embodiment.

### DESCRIPTION OF EXEMPLARY EMBODIMENTS

Hereinafter, an embodiment will be described. The embodiment described below does not unduly limit the contents of the invention set forth in the appended claims. Moreover, not all of the configurations described in the embodiment may necessarily be indispensable components of the invention.

### 1. Technique of Embodiment

First, a technique of the embodiment will be described. As described above, vital information measuring devices including various vital sensors mounted thereon have been known, and the number of vital sensors included in one vital information measuring device tends to increase. Moreover, like a vital information measuring device (wearable life support apparatus) disclosed in JP-A-2006-320735, portable and wearable vital information measuring devices have also been widely known.

In the portable and wearable vital information measuring device, vital information is measured over a long time in many cases. For example, when a change in heart rate during exercise such as jogging is measured, since it is assumed to continuously measure the heart rate from the start to the end of the exercise, the measurement is performed for about several tens minutes to several hours. It is sufficient for a stationary vital information measuring device (for example, a stationary sphygmomanometer) to measure vital information (a blood pressure value and information necessary for measuring the value) for about several tens seconds. Therefore, it can be said that a long time measurement is a great feature of portable devices. Moreover, the present applicant assumes, as one embodiment, a technique for acquiring a "life log" with a vital information measuring device worn by a user for a long time (continuously in a more limited sense) and by driving as many sensors as possible.

In this case, given that there are many vital sensors and there exists a demand for continuing the drive of the vital sensors or processes using vital information as long as possible, power management of the vital information measuring device is a big problem. As described above, since the vital information measuring device is assumed to be a portable and wearable device, a built-in battery or the like is used in many cases for the measurement of vital information without using commercial power (AC power or the like). Therefore, it is difficult to drive various sensors effectively and for a long time without performing power management in view of power saving.

JP-A-2006-320735 also discloses a technique for power saving, but the technique is limited to a simple one such as turning off a specific sensor in sleeping. However, while the number of vital sensors mounted on the vital information measuring device is increasing, vital information in which a user is really interested is limited to only a small portion of the vital information in many cases.

For example, it is sufficient for a diabetic patient to properly measure only a blood-sugar level, or it is sufficient for a patient with respiratory embarrassment to properly measure only an arterial oxygen saturation (SpO2). That is, for a user who is a diabetic patient and has a normal respiratory function, a blood-sugar level sensor (for example, a low-invasive blood-sugar level monitor using a change in electromagnetic field, light, or the like) desirably performs measurement frequently (constantly in a more limited sense) regardless of a battery state, but an arterial oxygen saturation sensor (pulse oximeter) may be brought into a low power consumption mode or an off state with less chance of problems under a situation of low remaining battery power or the like. Conversely, for a user who is a patient with respiratory embarrassment and does not have diabetes, a pulse oximeter desirably operates constantly, but a blood-sugar level monitor may be brought into the low power consumption mode or the off state depending on the situation.

That is, processing in view of how important each vital sensor is for the user, in other words, how high the priority to continue operating is is very useful in performing the power management of the various sensors provided in the vital information measuring device. However, a technique for setting the priority based on a measured value and a technique for performing the power management using the priority are not found in the related art.

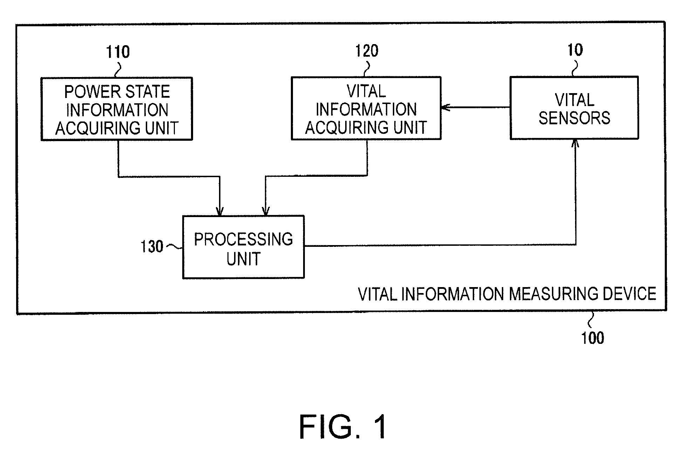

Therefore, the present applicant proposes a power management technique in which the information of priority is set for each of vital sensors and the priority is used. Specifically, a vital information measuring device 100 according to the embodiment includes, as shown in Fig. 1, a power state information acquiring unit 110 that acquires power state information indicating a power state, a vital information acquiring unit 120 that acquires a plurality of pieces of vital information from a plurality of vital sensors 10, and a processing unit 130 that performs power management of the plurality of vital sensors 10 based on the power state information. The processing unit 130 sets, based on each of the plurality of pieces of vital information, a priority (the information of priority) of power supply for each of the plurality of vital sensors, and executes the power management on the plurality of vital sensors 10 based on the priority and the power state information.

Here, the power state information is information indicating the state of power, and is, in a more limited sense, information indicating the remaining battery power of a battery included in the vital information measuring device 100. Alternatively, when the vital information measuring device 100 is a device that is operable in charging of a battery using AC power or the like, the power state information may be information indicating whether the vital information measuring device is operating with AC power or operating with a battery.

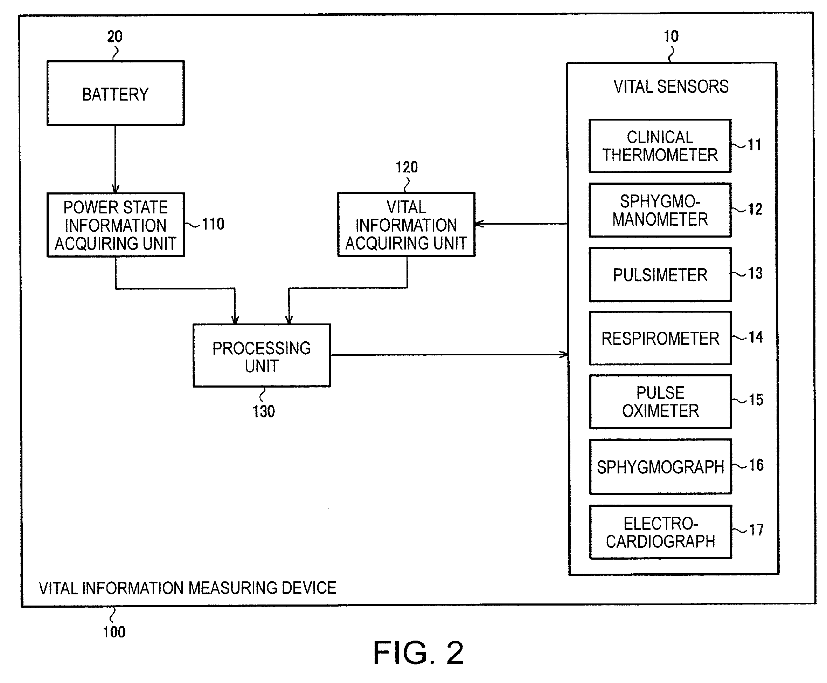

Moreover, the plurality of vital sensors 10 may include, as shown in Fig. 2, a clinical thermometer 11, a sphygmomanometer 12, a pulsimeter 13, a respirometer 14, a pulse oximeter 15, a sphygmograph 16, and an electrocardiograph 17. The clinical thermometer 11 is a sensor to measure a body temperature. The sphygmomanometer 12 is a sensor to measure a blood pressure. The pulsimeter 13 is a sensor to measure a pulse rate (heart rate). The respirometer 14 is a sensor to measure a respiratory flow rate, a respiratory resistance, or the like. The pulse oximeter 15 measures the arterial oxygen saturation (SpO2) as described above. The sphygmograph 16 is a sensor to measure a blood pressure in a peripheral vascular system or a change in the volume thereof caused by heart beats, and the measurement is usually performed at a fingertip to be used for the diagnosis of heart disease or peripheral arterial disease. The electrocardiograph 17, which records an electrocardiogram (ECG), records the electrical activity of the heart and is used for a test of abnormal heart activity such as arrhythmia. However, the vital sensors 10 are not limited to those shown in Fig. 1, and modifications can be implemented in which a portion of the sensors described above may be omitted or a sensor other than those described above, such as the blood-sugar level monitor described above, may be added. Moreover, although the vital sensors 10 are included in the vital information measuring device 100 in Fig. 1 or 2, this is not restrictive. The vital sensors 10 may be provided outside the vital information measuring device 100 (and at positions at which the vital sensors are worn by the user), and connected with the vital information measuring device 100 through a wired or wireless network.

The power management is, in a more limited sense, to perform management of power to be consumed in the operation of each of the vital sensors, in a process using vital information, and in each of processes performed in the vital information measuring device 100, In a more limited sense, the power management means to manage the operating state of the vital sensors 10. Moreover, the information of priority of power supply is information used when performing settings such as whether or not power is supplied to each of the vital sensors, or what level of supply power (operation rate of the vital sensor) is set when supplying power. The information of priority may be a priority as specific numerical value data. In this case, for example, a sensor having a high numerical value of priority is more preferentially supplied with power compared with a sensor having a low numerical value of priority. However, the information of priority is not limited to the priority represented by the numerical value data or the like, and may be other information as long as the information enables a priority comparison between the sensors. The information of priority is expressed simply as "priority" in the following description, but the "priority" can be extended to other information of priority.

By doing this, a priority can be set for each of the vital sensors using sensor information from each of the vital sensors, and the power management can be performed using the priority. Therefore, since the vital information from the vital sensors that are actually worn by a user is used, a vital sensor having high importance (priority) for the user can be determined based on a measured value, so that proper power management can be performed. The "proper power management" herein is power management in which, for example, a more important (high-priority) vital sensor is frequently operated while an unimportant vital sensor is set into the low power consumption mode or the off state.

In the embodiment, the power state information is used in the power management. Usually, if remaining battery power is sufficient, even when all of the vital sensors mounted are operated, it is unlikely that a problem such as running out of battery immediately occurs. Therefore, the power management in the embodiment is, in a more limited sense, a process of setting a low-priority vital sensor into the low power consumption mode or the off state when the remaining battery power is reduced. However, if a vital sensor is of very low importance for the user, the need to operate the vital sensor is low regardless of the power state. That is, even when the remaining battery power is sufficient, a predetermined vital sensor is not prevented from being set into the low power consumption mode or the off state. By doing such operation, the operating time of the vital information measuring device 100 can be prolonged. In light of the fact described above, the power management in the embodiment includes management in which power saving is achieved in a state of sufficient remaining battery power, and is not limited to management in which power saving is performed only when the remaining battery power is reduced.

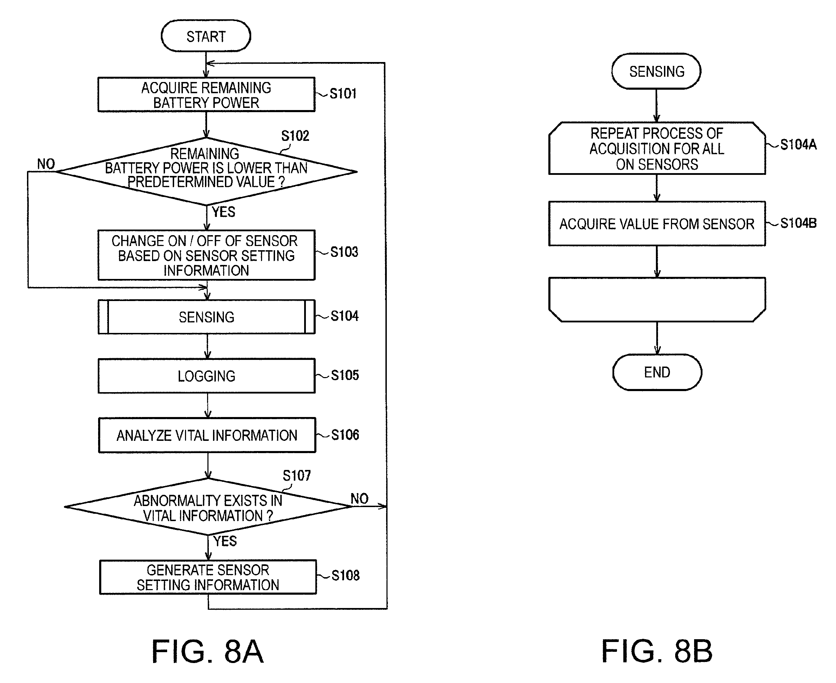

Configuration examples of the vital information measuring device 100 and a processing system 200 according to the embodiment will be described below. Thereafter, details of processes of the embodiment will be described using flowcharts in Figs. 8A and 8B and the like.

### 2. System Configuration Example

Fig. 2 shows a detailed configuration example of the vital information measuring device 100 according to the embodiment. As shown in Fig. 2, the vital information measuring device 100 includes the power state information acquiring unit 110, the vital information acquiring unit 120, the processing unit 130, the vital sensors 10, and a battery 20. However, the vital information measuring device 100 is not limited to the configuration in Fig. 2, and various modifications can be implemented in which a portion of the components may be omitted or another component may be added.

The functions of the processing unit 130 can be realized by hardware such as various processors (a CPU or the like) or an ASIC (a gate array or the like), or programs. Moreover, although not shown in Fig. 2, the vital information measuring device 100 may include a notifying unit that notifies a user of measured results of vital information and the like. Various modes of notification are conceivable in the notifying unit. Sounds or voices may be produced, a light emitting unit such as an LED may be lighted, or a vibrating unit may be vibrated. Moreover, the notifying unit may be realized by a display unit that displays various display screens. The display unit can be realized by a liquid crystal display, an organic EL display, or the like. Moreover, the vital information measuring device 100 may include a storing unit that stores measured vital information, or information such as the priority set in each of the vital sensors. The functions of the storing unit can be realized by a memory such as a RAM, an HDD (hard disk drive), or the like.

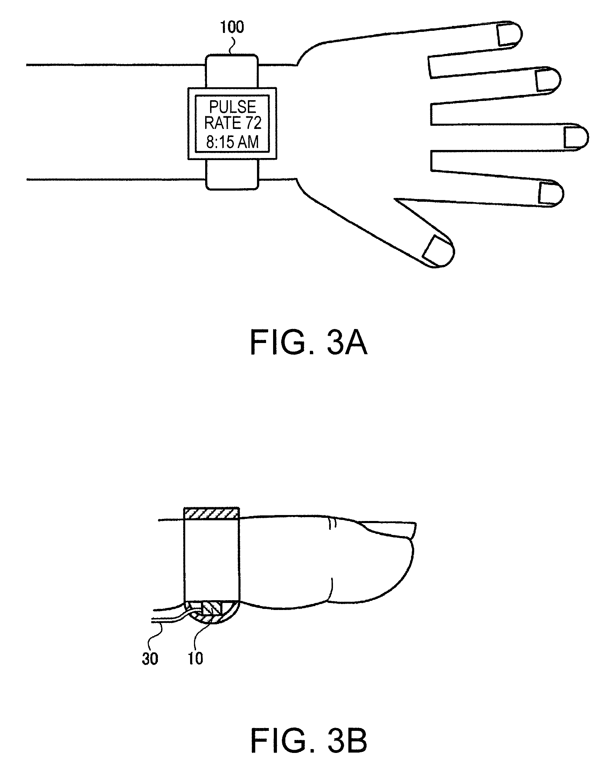

Various shapes are conceivable for the vital information measuring device 100. For example, as shown in Fig. 3A, the vital information measuring device 100 may be a band-type or wristwatch-type device that is wrist-worn by a user. Alternatively, as shown in Fig. 3B, the vital information measuring device 100 may be a finger-worn device although there are many limitations on the notifying unit such as a display unit, or the number of the vital sensors 10 to be mounted. Moreover, a band-type device and a finger-worn device may operate in conjunction with each other, in which the vital information measuring device 100 of the band-type shown in Fig. 3A is used together with a portion of the plurality of vital sensors 10 provided on the finger as shown in Fig. 3B. In this case, as shown in Fig. 3B, wired or wireless communication is performed between a plurality of devices such as the case where vital information from the vital sensor 10 is transmitted to the vital information measuring device 100 through a wired cable 30.

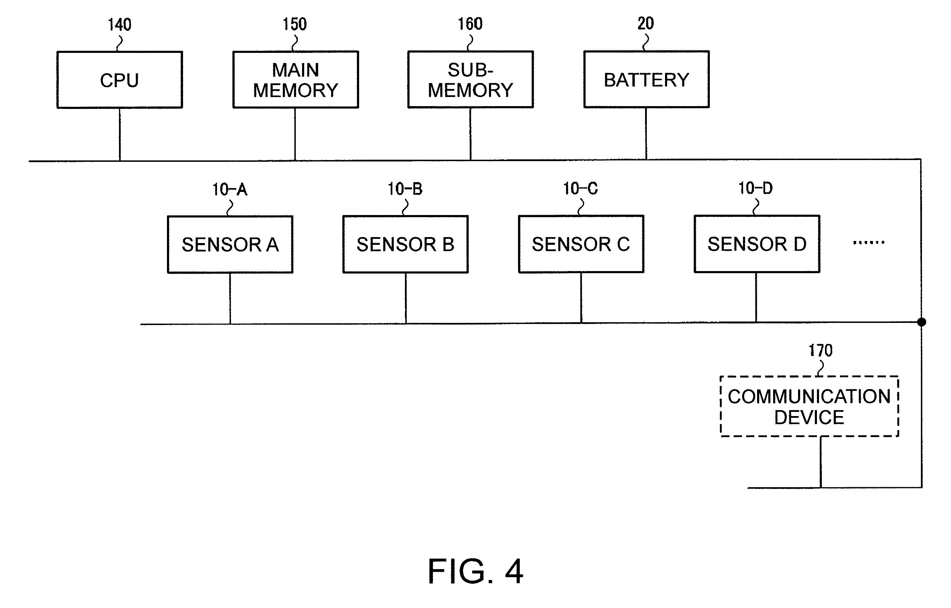

Fig. 4 shows a detailed configuration example of the vital information measuring device 100. The vital information measuring device 100 includes a CPU 140, a main memory 150, a sub-memory 160 , the battery 20, and sensors A to D (10-A to 10-D). Here, the processing unit 130 described above may be realized by the CPU 140. The storing unit described above (not shown) may be realized by the main memory 150 and the sub-memory 160. The sensor A or the like is a vital sensor that acquires some kind of vital information, and may be, for example, any of the vital sensors shown in Fig. 2. The vital information measuring device 100 may include a sensor that senses information that is not sorted into vital information. For example, the vital information measuring device 100 may include an acceleration sensor, a gyro sensor, and a terrestrial magnetism sensor. Moreover, the vital information measuring device 100 may include a communication device 170. The communication device 170 performs communication with another device. For example, when vital information acquired by the vital sensors 10 is logged over a long period, or advice information on the health of the user is generated based on the vital information, a server system or the like with a large storage capacity and high processing speed is used in some cases. In such a case, the communication device 170 transmits the vital information acquired by the vital sensors 10 to the server system or the like.

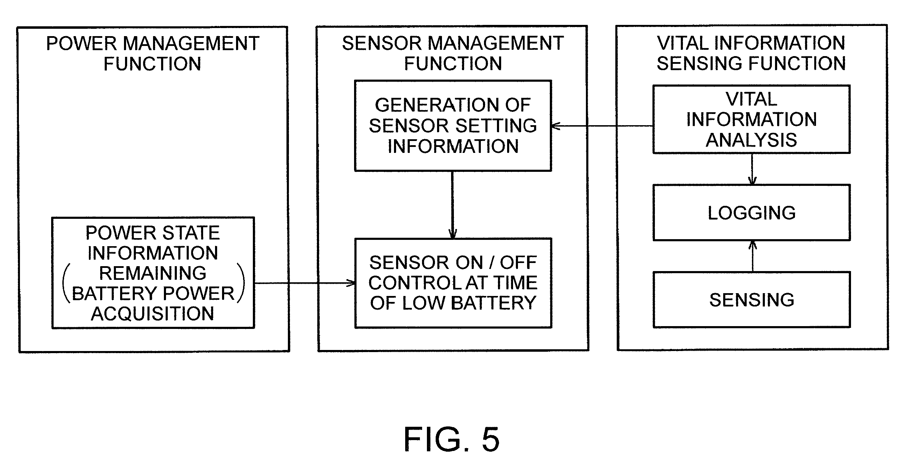

Fig. 5 shows a functional block diagram of the vital information measuring device 100. The vital information measuring device 100 includes a power management function, a sensor management function, and a vital information sensing function. The vital information sensing function performs sensing of vital information, logging of the sensed vital information, and analysis of the vital information using the log data. The power management function acquires power state information (in a more limited sense, remaining battery power), and transmits the information to the sensor management function. The sensor management function generates sensor setting information based on the analysis results of the vital information. The sensor setting information herein is, for example, the information of priority described above. Then, based on the sensor setting information and the power state information, control such as on/off of the sensors is performed when the remaining battery power is reduced.

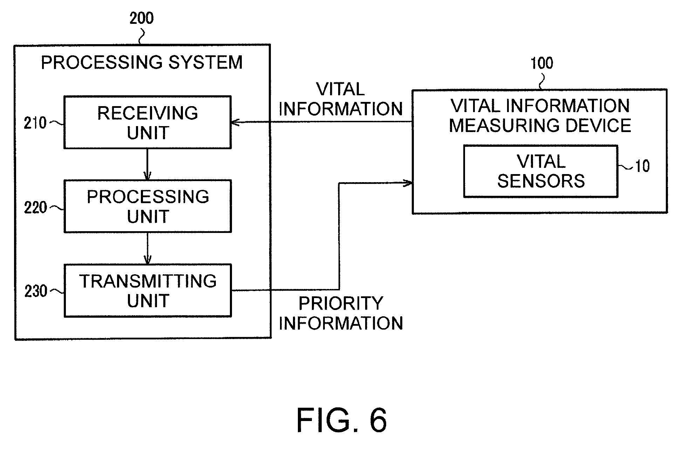

As shown in Fig. 6, the technique of the embodiment can be applied to the processing system 200 including a receiving unit 210 that receives the plurality of pieces of vital information from the plurality of vital sensors 10 provided in the vital information measuring device 100, a processing unit 220 that produces priority information used for the power management of the vital information measuring device 100, and a transmitting unit 230 that transmits the priority information to the vital information measuring device. The processing unit 220 of the processing system 200 generates the priority information for setting the priority of power supply for each of the plurality of vital sensors based on each of the plurality of pieces of vital information.

By doing this, it is sufficient for the vital information measuring device 100 to acquire information from the vital sensors 10 and transmit the information, and another device can generate the priority information for power management. In this case, since the vital information measuring device 100 operates the vital sensors 10 according to the priority information transmitted from the processing system 200, the configuration of the vital information measuring device 100 can be simplified, so that power consumption can be reduced. The priority information herein may be the above-described priority itself, or may be other information for setting the priority.

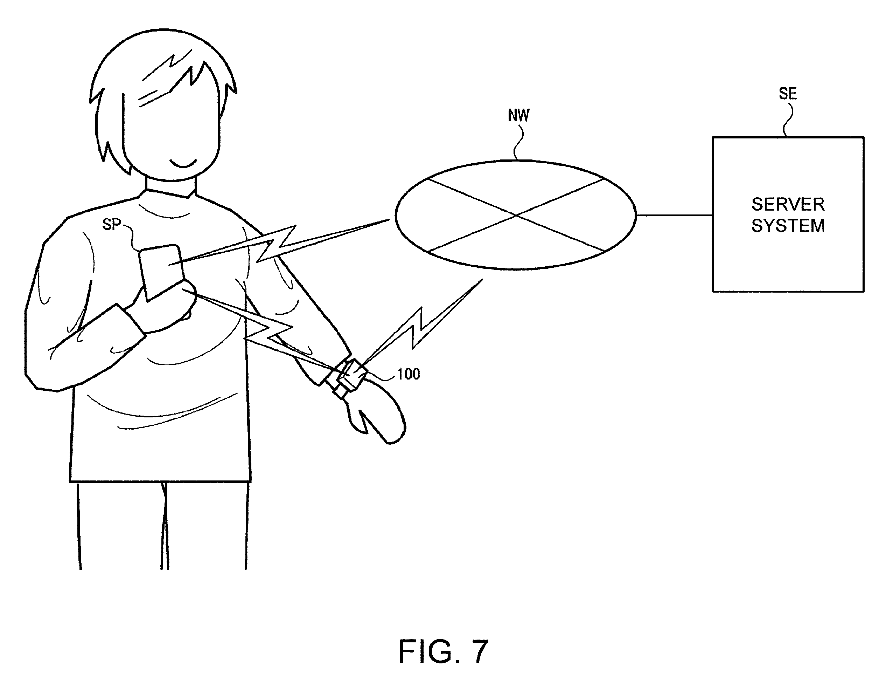

Various types are conceivable for a device including the processing system 200 in Fig. 6. For example, as shown in Fig. 7 , when the user wearing the vital information measuring device 100 has a portable terminal device SP such as a smartphone, the processing system 200 may be included in the portable terminal device SP. In this case, a network NW such as the Internet, or a short-range wireless communication may be used for communication between the vital information measuring device 100 and the portable terminal device SP.

Alternatively, the processing system 200 in Fig. 6 may be included in a server system SE (cloud system) connected with the vital information measuring device 100 via the network NW. By doing this, since scalable computing resources or storing devices of the cloud system can be used, more advanced and flexible processing can be realized without being bounded by limited computing resources or storing device capacity of a wearable device.

For example, by accumulating vital information for a long period such as several years or dozen years or so, and using the long-period data, more advanced processing not possible with the wearable vital information measuring device 100 alone can be performed. For example, even in such a case where "although quite well lately, the user often suffered from arrhythmia until a decade ago", it is possible, by accumulating past vital information and making determination, to generate priority information such as "setting the priority of electrocardiograph also high by way of precaution".

Moreover, it is easy to update the analysis process of vital information to one having higher accuracy and high added value. In the case of the configuration of the wearable vital information measuring device 100 alone, the user needs, for updating the analysis process (updating algorithms of the analysis process, or software for performing the analysis process), to take a plurality of steps: first, temporarily stopping the operation of the device; second, performing an operation of connecting with the network; third, instructing the updating process of the analysis process; and fourth, waiting until the completion of the updating. However, this requires time and effort from the user, and sensing of vital information cannot be performed during the updating process.

In contrast, the use of the cloud system enables the updating of the analysis process without stopping the system, and therefore, interruption of sensing can be suppressed. Moreover, the user does not need to spend time and effort for updating, and can use the analysis process whose processing contents are sophisticated (becomes intelligent) without awareness.

Various communication routes are conceivable between the vital information measuring device 100 and the server system SE. For example, when the vital information measuring device 100 can be directly connected to the network NW, the vital information measuring device 100 may directly communicate with the server system SE via the network NW. Alternatively, the vital information measuring device 100 may communicate with the server system SE via another device such that the vital information measuring device 100 first transmits vital information to the portable terminal device SP using short-range wireless communication or the like, and the portable terminal device SP transfers the vital information to the server system SE via the network NW.

### 3. Details of Processes

Next, details of the processes of the embodiment will be described according to flowcharts in Figs. 8A and 8B. As shown in Fig. 8A, when the process of the embodiment is started, remaining battery power (in a broad sense, power state information) is first acquired (S101). Then, it is determined whether or not the remaining battery power is lower than a predetermined threshold value (S102).

When the remaining battery power is lower than the predetermined value (Yes at S102), the on/off or the power consumption mode of the vital sensors 10 is changed based on sensor setting information (priority) (S103). Specifically, when it is determined based on the power state information that the remaining battery power is lower than the predetermined value, the processing unit 130 performs power management in which a high-priority vital sensor is set into a normal power consumption mode and a low-priority vital sensor is set into a low power consumption mode or the off state. Here, the low power consumption mode is an operation mode in which power consumption is low compared to the normal power consumption mode, and is, for example, an operation mode in which an operation rate (the number of times of acquisition of sensor information per unit time) of the vital sensor is low compared to the normal power consumption mode. By doing this, when the remaining battery power is low, that is, when a reduction in the power consumption of the vital information measuring device 100 is strongly required, it is possible to reduce the power consumption of the vital sensor whose priority is low and that is determined as being less important for the user. Also in that case, since the vital sensor whose priority is high and whose continuous measurement is determined as being important for the user continues to operate, it is possible to suppress the likelihood that necessary information cannot be acquired due to power saving. After the process at S103, sensing is performed by each of the vital sensors (S104).

On the other hand, when the remaining battery power is equal to or higher than the predetermined value (No at S102), there is less need to reduce power consumption, and therefore, sensing is performed without performing the process at S103 (S104).

Fig. 8B is a flowchart showing details of S104, in which a process of acquiring a signal value (vital information) from the vital sensor (S104B) is performed on all of the sensors that are in the on state (S104A). As described above, a portion of the vital sensors is in the off state depending on the situation of the remaining battery power, so that the acquisition of vital information is, of course, not performed on the vital sensor that is in the off state as shown in Fig. 8B. Although only the on or off state of the vital sensors is shown in Figs. 8A and 8B, power consumption may be reduced by changing the operation rate as described above. In that case, sensing in consideration of the operation rate may be performed at S104. For example, a process may be performed such that 10 sensor values are acquired from the vital sensor in the normal power consumption mode when the step at S104 is performed 10 times while 5 sensor values are acquired from the vital sensor in the low power consumption mode in which the operation rate is 1/2 that of the normal power consumption mode when the step at S104 is performed 10 times. That is, the process in Fig. 8B can be extended to a flow in which when the vital sensor is in the on state or the low power consumption mode and it is value acquiring timing right now, the value is acquired, while when the vital sensor is in the off state or the low power consumption mode and it is not value acquiring timing right now, the acquisition of the value is skipped. For this point, various modifications can be implemented.

After performing sensing, the sensing results are logged (S105), and the analysis of vital information is performed using the log data (S106). Then, it is determined whether or not an abnormality exists in the vital information (S107). When the abnormality exists, sensor setting information is generated (priority is set) or updated (S108). Specifically, the processing unit 130 may perform, based on the plurality of pieces of vital information, a process of determining whether or not an abnormal value is detected in the plurality of vital sensors. When the abnormal value is detected in a first vital sensor and the abnormal value is not detected in a second vital sensor that is different from the first vital sensor in the plurality of vital sensors, a priority of the first vital sensor is set higher than a priority of the second vital sensor.

By doing this, the priority of the vital sensor can be set based on the detection or non-detection of the abnormal value. Specifically, when the abnormal value is detected, a situation such as suspicion of a disorder relating to the vital sensor is assumed for the user. Therefore, vital information from the vital sensor is to be frequently acquired to closely observe the vital information. On the other hand, when the abnormal value is not detected, the user has less potential for the disorder relating to the vital sensor. Therefore, a problem is less likely to occur even when the vital information from the vital sensor cannot be acquired (or an acquisition rate is lowered). However, the "abnormal value" in the embodiment does not need to be determined based on whether or not a disorder is suspected. A numerical value range in which the need to closely observe vital information is low is determined as "normal", while when a value exceeds the normal numerical value range, it is determined that the abnormal value is detected. For example, it is also conceivable, in a situation in which the failure of the vital sensor is suspected, that whether or not the vital sensor has an actual failure is determined by analyzing vital information (sensor value) . In that case, "normal" or "abnormal" is determined so as not to bring the vital sensor suspected of a failure into the off state or the low power consumption mode. For example, a numerical value range of the sensor value assumed when a normal human wears the device (in this case, both a numerical value of a healthy user and a numerical value of a user suspected of a disorder are included) may be determined as "normal", and the other values may be determined as "abnormal".

After the process at S108, or when the abnormality does not exist in the vital information (No at S107), the flow returns to S101, and the process continues.

In the above description, the determining technique based on the power state information is a determination whether or not the remaining battery power is higher than the predetermined value, the setting technique of priority is based on the detection or non-detection of the abnormal value, and the operation control technique of the vital sensor is performed such that when the priority is high, the vital sensor is brought into the normal power consumption mode, while when the priority is low, the vital sensor is brought into the low power consumption mode or the off state. However, various modifications can be implemented for each of the techniques.

For example, as described above, even when the remaining battery power is sufficient, not all of the vital sensors may be brought into the normal power consumption mode, but a portion of the vital sensors may be brought into the low power consumption mode or the off state.

Moreover, the level of priority is not limited to two levels, high and low, and three or more levels may be used. For example, setting is possible such that 0 to N (N is an integer of 2 or more) integers are used as priorities in which the priority is high as the value is higher. Alternatively, there is no need to limit the value to an integer, and an interval between values may be more finely set (close to continuous values). In that case, it may be determined not only whether or not the abnormal value is detected, but also how far the abnormal value exceeds the normal numerical value range in the case of abnormality. For example, when the normal numerical value range of vital information is from 20 to 80, it is apparent in comparison between 81 and 150 that 150 is highly abnormal. That is, in comparison between a priority P₈₁ that is set when a sensor value is 81 and a priority P₁₅₀ that is set when a sensor value is 150, the determination process of the abnormal value and the setting process of the priority are performed so as to satisfy the relationship: P₁₅₀ > P₈₁.

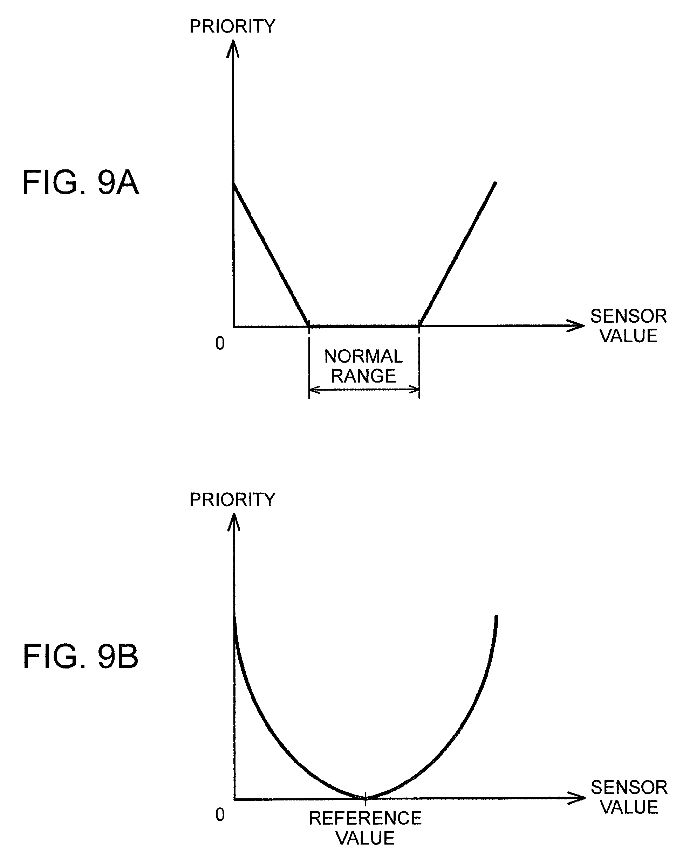

Alternatively, a function that associates a sensor value with a priority may be prepared for each of the vital sensors. For example, as shown in Fig. 9A, a function may be used by which the value of priority is low (for example, 0) for the sensor value within the normal range while the priority is high as the sensor value is higher than the upper limit of the normal range or the sensor value is lower than the lower limit of the normal range. Moreover, the priority may be changed according to a numerical value within the normal range. For example, a function shown in Fig. 9B may be used. When the function in Fig. 9B is used, the process does not change depending on whether a sensor value falls within the normal range or outside the normal range. That is, in one modified example of the embodiment, the priority may be set based on vital information without determining the detection or non-detection of the abnormal value.

Moreover, Fig. 9A or 9B shows a graph that is bilaterally symmetrical, but this is not restrictive. For example, it is also conceivable that a sensor value that is lower than the lower limit of the normal range indicates a less serious situation, but a sensor value that is higher than the upper limit of the normal range indicates a very serious situation. In that case, a function may be used by which the gradient or shape of a graph is varied depending on whether a sensor value is higher than the upper limit of the normal range or lower than the lower limit.

Also as to the remaining battery power, the process does not need to be performed based on two levels of whether or not the remaining battery power is lower than the predetermined value, and the process may be performed based on three levels or more. For example, when, in the plurality of vital sensors, a first priority is set for the first vital sensor, a second priority that is low compared to the first priority is set for the second vital sensor, and a third priority that is low compared to the second priority is set for a third vital sensor, the processing unit 130 sets the first vital sensor, the second vital sensor, and the third vital sensor into the normal power consumption mode in a first power state, sets the first vital sensor and the second vital sensor into the normal power consumption mode and sets the third vital sensor into the low power consumption mode or the off state in a second power state in which the remaining battery power is low compared to the first power state, and sets the first vital sensor into the normal power consumption mode and sets the second vital sensor and the third vital sensor into the low power consumption mode or the off state in a third power state in which the remaining battery power is low compared to the second power state.

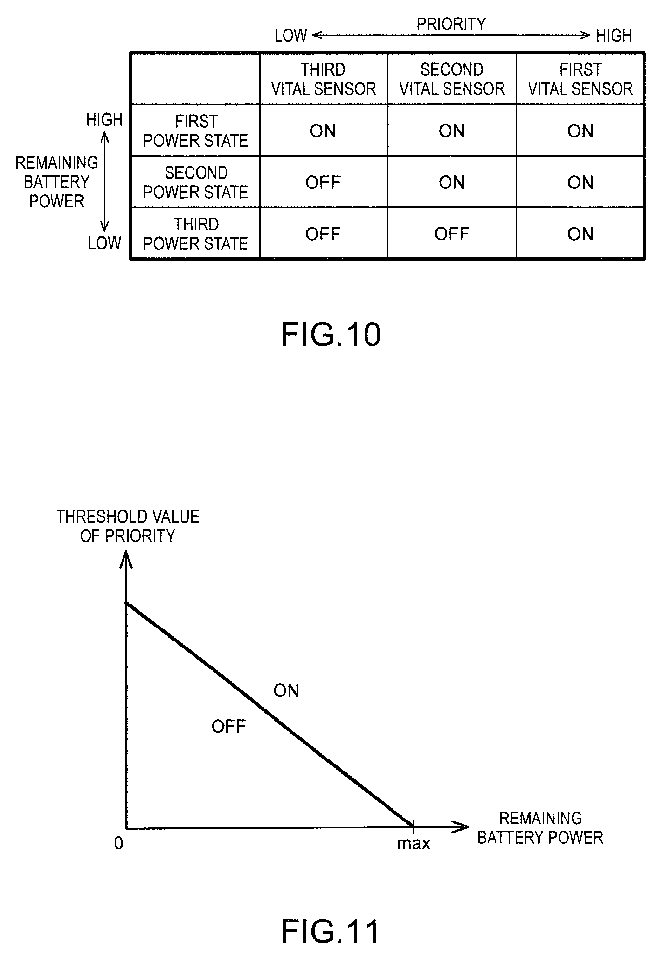

This is shown in Fig. 10. The state is indicated by ON/OFF in Fig. 10, but ON can be extended to the normal power consumption mode, and OFF can be extended to the low power consumption mode or the off state, as described above. By doing this, also as to the power state, power management can be performed by setting three or more levels. Specifically, as the remaining battery power is higher, the number of vital sensors that remain in the on state is large. Conversely, as the remaining battery power is lower, the number of vital sensors that remain in the on state is reduced to increase the number of vital sensors that are brought into the off state or the like because power saving is strongly requested.

Moreover, by extending the technique in Fig. 10, a threshold value of priority serving as a boundary between the on and off states can be set according to the power state (remaining battery power). Specifically, the processing unit 130 sets the threshold value of priority based on power state information; and then, the processing unit 130 sets a vital sensor whose set priority is higher than the threshold value in the plurality of vital sensors into the normal power consumption mode, and sets a vital sensor whose set priority is equal to or lower than the threshold value in the plurality of vital sensors into the low power consumption mode or the off state.

Fig. 11 shows an example of this process. As shown in Fig. 11, as the remaining battery power is higher, the threshold value is set low. By doing this, since the threshold value of priority is low in a state where the remaining battery power is high, the number of vital sensors whose set priority is higher than the threshold value, that is, the number of sensors that are set into the on state (the normal power consumption mode) is increased. Conversely, since the threshold value of priority is high in a state where the remaining battery power is low, the number of vital sensors whose set priority is equal to or lower than the threshold value, that is, the number of sensors that are set into the off state (the low power consumption mode) is increased. Therefore, proper sensor control is possible according to the remaining battery power.

In Fig. 11, the threshold value is continuously and linearly changed with respect to a change in remaining battery power, but this is not restrictive. Various modifications can be implemented such as changing the threshold value in a stepwise manner (in a discrete manner) as in the example in Fig. 10.

Moreover, the process may be performed based on the number of vital sensors that are set into the on state (the normal power consumption mode). For example, when there are many vital sensors whose priority is set very high, if the operation of each of the vital sensors is determined using the threshold value in Fig. 11 or the like, there are many sensors that are set into the on state regardless of low remaining battery power, and therefore, it is conceivable that an effect of reducing power consumption is insufficient. Conversely, when the priorities of all of the vital sensors are set low, it is conceivable that there is no sensor that operates at the time of low remaining battery power and excessive power saving occurs. That is, in this power state, a condition such that the number of vital sensors that are turned on is M (M is a positive integer) may be added.

For example, as a result of performing the threshold value determination as in Fig. 11, when the number of sensors that are turned on is greater than M, M pieces of the sensors are selected as operation targets. Alternatively, in the setting step of priority, the number of sensors whose priority is equal to or higher than a given value (in a more limited sense, a threshold value to be set) may be limited to M. In this case, the priority to be set for each of the vital sensors is determined not only by vital information from the vital sensor but set also using the information of priority set for the other vital sensors (in a broad sense, vital information from the other vital sensors, or information obtained based on the vital information).

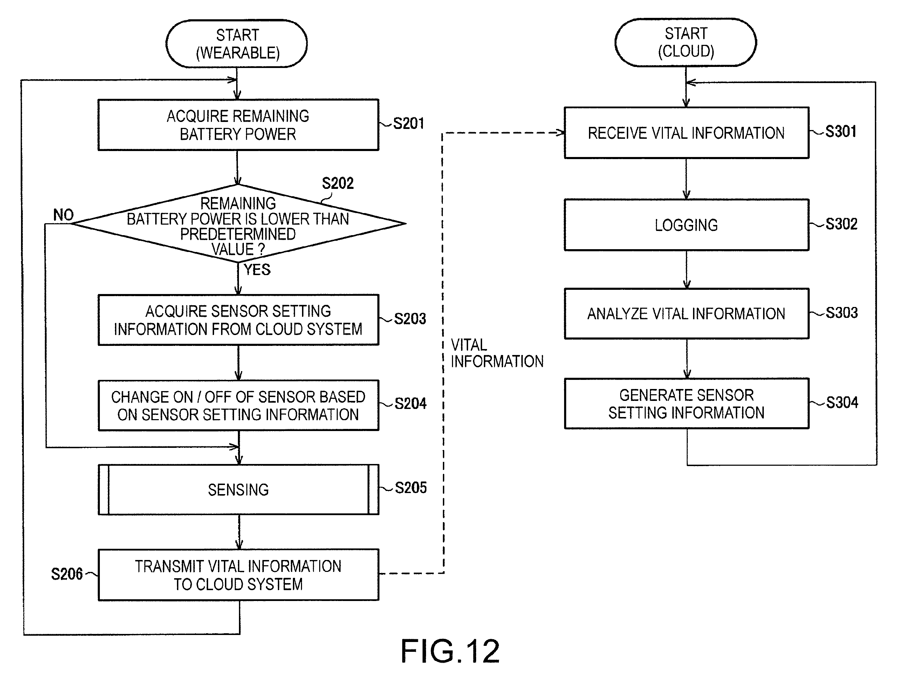

Although an example in which the power management is performed in the vital information measuring device 100 has been described above, the priority information for the power management may be generated in the server system SE or the like as described above using Fig. 7 or the like. A flow of a process in this case will be described using a flowchart in Fig. 12.

When this process is started, the remaining battery power of the vital information measuring device 100 is first acquired in the vital information measuring device 100 (wearable device) (S201). Then, it is determined whether or not the remaining battery power is lower than the predetermined value (S202). When the remaining battery power is equal to or higher than the predetermined value, sensing is performed (S205), and vital information as the sensing result is transmitted to the cloud system (the server system SE or the like) (S206). Here, the sensing process at S205 is similar to the process in Fig. 8B.

The cloud system receives the vital information transmitted at S206 (S301), and logs the received vital information (S302). Further, the cloud system performs the analysis of the vital information using the log data (S303), and generates the information of priority (sensor setting information) based on the analysis result (S304). After the process at S304, the cloud system waits for the transmission of vital information from the vital information measuring device 100. When the transmission is performed, the flow returns to S301.

When the remaining battery power is lower than the predetermined value in the vital information measuring device 100 (Yes at S202), the sensor setting information is acquired from the cloud system (S203), and control such as the on/off of the vital sensors is performed based on the acquired sensor setting information (S204). At S203, a request for the sensor setting information generated at S304 is made to the cloud system, and the sensor setting information is received as a response of the request. After the process at S204, the flow proceeds to S205, and sensing is performed.

The processes of the vital information measuring device 100, the processing system 200, and the like of the embodiment may be partially or largely realized by a program. In this case, a processor such as a CPU executes the program to thereby realize the vital information measuring device 100, the processing system 200, and the like of the embodiment. Specifically, a program stored in a non-transitory information storage medium is read, and the processor such as a CPU executes the read program. Here, the information storage medium (computer-readable medium) stores programs, data, and the like, and the functions of the information storage medium can be realized by an optical disc (a DVD, a CD, or the like), an HDD (hard disk drive), a memory (a card memory, a ROM, or the like) or the like. Then, the processor such as a CPU performs the various processes of the embodiment based on the program (data) stored in the information storage medium. That is, in the information storage medium, a program (program for causing a computer to execute the processes of each part) causing a computer (a device including an operating unit, a processing unit, a storing unit, and an output unit) to function as each part of the embodiment is stored.

Moreover, the vital information measuring device 100, the processing system 200, and the like of the embodiment may include a processor and a memory. The processor herein may be, for example, a CPU (Central Processing Unit). However, the processor is not limited to a CPU, and various processors such as a GPU (Graphics Processing Unit) or a DSP (Digital Signal Processor) can be used. Moreover, the processor may be a hardware circuit using an ASIC. The memory stores a computer-readable command. The command is executed by the processor, so that each part of each of the vital information measuring device 100, the processing system 200, and the like according to the embodiment is realized. The memory herein may be a semiconductor memory such as an SRAM or a DRAM, a register, a hard disk, or the like. The command herein may be a command of a command set constituting a program, or a command to instruct the hardware circuit of the processor to operate.

While the embodiment has been described above in detail, it should be easily understood by those skilled in the art that many modifications not substantially departing from the novel matters and advantages of the invention are possible. Accordingly, all of those modified examples are deemed to be included in the scope of the invention. For example, the terms mentioned in the specification or the drawings at least once together with different terms in a broader sense or a similar sense may be replaced with the different terms in any part of the specification or the drawings. Moreover, the configurations and operations of the vital information measuring device 100, the processing system 200, and the like are not limited to those described in the embodiment, and various modifications can be implemented.

## Claims

1. A vital information measuring device comprising:
a power state information acquiring unit that acquires power state information indicating a power state;
a vital information acquiring unit that acquires a plurality of pieces of vital information from a plurality of vital sensors; and
a processing unit that performs power management of the plurality of vital sensors based on the power state information, wherein
the processing unit sets a priority of power supply for each of the plurality of vital sensors based on each of the plurality of pieces of vital information, and executes the power management on the plurality of vital sensors based on the priority and the power state information.

2. The vital information measuring device according to claim 1, wherein
the processing unit performs, based on the plurality of pieces of vital information, a process of determining whether or not an abnormal value is detected in the plurality of vital sensors, and
when the abnormal value is detected in a first vital sensor and the abnormal value is not detected in a second vital sensor that is different from the first sensor in the plurality of vital sensors, the processing unit sets the priority of the first vital sensor higher than the priority of the second vital sensor.

3. The vital information measuring device according to claim 1, wherein
when it is determined based on the power state information that remaining battery power is equal to or lower than a predetermined value, the processing unit performs the power management in which the vital sensor whose priority is high is set into a normal power consumption mode and the vital sensor whose priority is low is set into a low power consumption mode or an off state.

4. The vital information measuring device according to claim 1, wherein
when, in the plurality of vital sensors, a first priority is set for a first vital sensor, a second priority that is low compared to the first priority is set for a second vital sensor, and a third priority that is low compared to the second priority is set for a third vital sensor, the processing unit sets
the first vital sensor, the second vital sensor, and the third vital sensor into a normal power consumption mode in a first power state,
sets the first vital sensor and the second vital sensor into the normal power consumption mode and sets the third vital sensor into a low power consumption mode or an off state in a second power state in which remaining battery power is low compared to the first power state, and
sets the first vital sensor into the normal power consumption mode and sets the second vital sensor and the third vital sensor into the low power consumption mode or the off state in a third power state in which the remaining battery power is low compared to the second power state.

5. The vital information measuring device according to claim 1, wherein
the processing unit sets
a threshold value of the priority based on the power state information,
sets the vital sensor whose set priority is higher than the threshold value in the plurality of vital sensors into a normal power consumption mode, and
sets the vital sensor whose set priority is equal to or lower than the threshold value in the plurality of vital sensors into a low power consumption mode or an off state.

6. A processing system comprising:
a receiving unit that receives a plurality of pieces of vital information from a plurality of vital sensors provided in a vital information measuring device;
a processing unit that produces priority information used for power management of the vital information measuring device; and
a transmitting unit that transmits the priority information to the vital information measuring device, wherein
the processing unit generates, based on each of the plurality of pieces of vital information, the priority information for setting a priority of power supply for each of the plurality of vital sensors.

7. A vital information processing method comprising:
receiving a plurality of pieces of vital information from a plurality of vital sensors provided in a vital information measuring device;
generating, based on each of the plurality of pieces of vital information, priority information used for power management of the vital information measuring device and for setting a priority of power supply for each of the plurality of vital sensors; and
transmitting the priority information to the vital information measuring device.

8. A program causing a computer to function as:
a receiving unit that receives a plurality of pieces of vital information from a plurality of vital sensors provided in a vital information measuring device;
a processing unit that produces priority information used for power management of the vital information measuring device; and
a transmitting unit that transmits the priority information to the vital information measuring device, wherein
the processing unit generates, based on each of the plurality of pieces of vital information, the priority information for setting a priority of power supply for each of the plurality of vital sensors.
